(19) Europäisches Patentamt
European Patent Office
Office européen des brevets

(11) Numéro de publication: **0 261 017 B1**

(12) # FASCICULE DE BREVET EUROPEEN

(45) Date de publication de fascicule du brevet: **20.03.91**

(51) Int. Cl.$^5$: **C07C 229/06**, C07C 229/30, C07D 207/28, C07C 233/45, A61K 31/215, A61K 31/40

(21) Numéro de dépôt: **87401986.2**

(22) Date de dépôt: **04.09.87**

(54) **Esters d'aminoacides d'alcools cycloaliphatiques, procédé de préparation et utilisation comme médicaments.**

(30) Priorité: **16.09.86 FR 8612915**

(43) Date de publication de la demande: **23.03.88 Bulletin 88/12**

(45) Mention de la délivrance du brevet: **20.03.91 Bulletin 91/12**

(84) Etats contractants désignés: **AT BE CH DE ES FR GB GR IT LI LU NL SE**

(56) Documents cités:
**EP-A- 0 154 472**
**FR-A- 1 510 466**
**GB-A- 1 156 389**
**GB-A- 1 464 985**
**DE-C- 261 228**
**GB-A- 729 550**
**GB-A- 1 436 329**
**NL-C- 75 913**

**TETRAHEDRON, vol. 40, no. 2, 1984, Pergamon Press, Ltd., Oxford (GB); M.JULIA et al.: "Etude cinétique de l'aminolyse en milieu non aqueux d'esters modèles de peptidyl-t-ARN; influence d'un groupe hydroxyle voisin", pp. 327-337**

(73) Titulaire: **PANMEDICA S.A.**
**1ère avenue-2065 M - L.I.D.**
**F-06516 Carros(FR)**

(72) Inventeur: **Laruelle, Claude**
**Avenue Bellevue**
**F-06270 Villeneuve Loubet(FR)**
Inventeur: **Lepant, Marcel**
**L'Escoundu Allée Clairefontaine**
**F-06140 Vence(FR)**
Inventeur: **Raynier, Bernard**
**9 Chemin du Malvan**
**F-06800 Cagnes(FR)**

(74) Mandataire: **Orès, Bernard et al**
**Cabinet ORES 6, Avenue de Messine**
**F-75008 Paris(FR)**

**EP 0 261 017 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

Rank Xerox (UK) Business Services

CHEMICAL ABSTRACTS, vol. 84, no. 11, 15 mars 1976, p. 489, no. 74612g, Columbus, Ohio, US; & JP-A-75 93 920

CHEMICAL ABSTRACTS, vol. 94, no. 10, 9 mars 1981, Columbus, OH (US); I.ABE et al.: "Resolution of amino acid enantiomers by glass capillary gas chromatography on easily prepared optically active stationary phases", p. 419, no. 72016y

CHEMICAL ABSTRACTS, vol. 105, no. 5, 4 août 1986, Columbus, OH (US); p. 703, no. 42457w; & JP-A-60 243073

CHEMICAL ABSTRACTS, vol. 84, no. 7, 16 février 1976, Columbus, OH (US); R.GOLD et al.: "Specificity of porcine elastase", p. 171, no. 40166z

## Description

La présente invention concerne des esters d'aminoacides naturels d'alcools cycloaliphatiques dont le cycle comprend 5 ou 6 chainons hydrocarbonés, éventuellement substitués par des radicaux hydrocarbonés à un ou deux atomes de carbone et ayant une activité hypolipémiante, leur procédé de fabrication et les compositions contenant de tels composés capables de corriger les dérèglements de la lipidémie. Ces produits correspondent à la formule générale suivante (I):

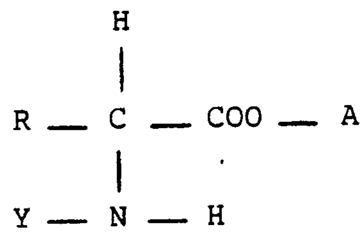

dans laquelle :
R représente le reste d'un α aminoacide naturel ou d'un dérivé simple de ce dernier dans le cas des aminoacides dicarboxyliques, tel que l'ester methylique, l'ester éthylique ou un amide primaire, Y est l'hydrogène, un radical acétyle, propionyle, benzoyle,
ou bien représente avec R un cycle pyrrolidinone 2 déterminant dans la formule générale un ester pyroglutamique. A représente un motif cycloaliphatique choisi dans le groupe composé de cyclohexyle, cyclohéxenyle, cyclopentyle, cyclopentenyle toujours substitué par un ou plusieurs radicaux choisis parmi les groupes : méthyle, éthyle, méthylène, éthylène, éthényle.

Selon un mode de réalisation avantageux desdits esters, le motif A représente un groupement cyclohexyle, substitué par un ou plusieurs radicaux méthyles.

Selon un autre mode de réalisation avantageux desdits esters, le motif A est un groupement cyclopentyle, substitué par un ou plusieurs radicaux méthyles. Conformément à l'invention, l'acide aminé est avantageusement un acide pyroglutamique.

L'étude de la synthèse des esters d'aminoacides ainsi que leurs propriétés physico-chimiques s'est longtemps limitée aux alcools des premiers termes, principalement méthyle et éthyle, ou aux esters benzyliques, et divers phénols substitués. Les premiers esters d'alkanols inférieurs ont fait en outre l'objet d'études pharmacologiques alors que les esters benzyliques ou phényliques étaient synthétisés comme intermédiaires de synthèse pour leur réactivité chimique particulière, par exemple les esters benzyliques pouvant être soumis à l'hydrogénolyse ou les esters pentachlorophényliques pour leurs réactivités chimiques.

L'intérêt des esters d'aminoacides d'alcools cycloaliphatiques a été souligné par le travail de HIROYUKI YAMAMOTO (Biopolymers 9. 41/52 - 1970) qui prépare les monomères γ menthyl glutamates et met à profit les propriétés du γ ester glutamique pour le soumettre à la polymérisation.

AJINOMOTO dans son brevet US 3 899 585 revendiquait les esters d'alkyl linéaires de C12 à C16 de l'acide pyroglutamique et leur activité bactéricide. Les propriétés physiques de ces mêmes esters de l'acide pyroglutamique étaient mises à profit par K. THOMAE dans son brevet allemand DE 2 102 172 en tant qu'adjuvants pour crèmes et lotions.

UNILEVER dans son addition française n° 2357544 revendiquait enfin les propriétés topiques adoucissantes notamment vis à vis des coups de soleil, du pyroglutamate du menthol.

La demanderesse a trouvé de façon surprenante que les esters d'aminoacides d'alcools cycloaliphatiques de structure particulière , présentaient de très intéressantes activiés pharmacologiques quand ils étaient administrés par voie générale.

Ces activités se manifestent notamment par la correction des dérèglements de la lipidémie,par l'abaissement du cholestérol circulant dans le cas d'hypercholestérolémies, le mécanisme étant dû à l'action inhibitrice vis à vis de l'Hydroxyméthyl glutarique coenzyme A réductase et pour certains à l'inhibition partielle de l'acétyl coenzyme acyl transférase.

Par aminoacides naturels, on comprend les alpha aminoacides constitutifs des protéines, ceux-ci comportent donc un site chiral en α déterminant des isomères -L- et -D-, l'isomère -L- étant toutefois le plus universellement répandu dans la nature. Dans certains cas, on comprendra la forme racémique de l'aminoacide.

On comprend également dans les termes aminoacides les dérivés les plus simples comme les mono

esters d'alkyle inférieur des acides aminés di-carboxyliques ou les dérivés N acétyle, N propionyle, N benzoyle sur l'azote porté par le carbone en position α.

On peut citer parmi les amino acides les plus courants : la glycine, l'alanine, la valine, la leucine, la proline, la thréonine, la sérine, la phényl-alanine, la cystéine, la cystine, la tyrosine, l'acide aspartique, l'asparagine, l'acide glutamique, la glutamine, l'acide pyroglutamique, le tryptophane, l'hydroxy-5 tryptophane, l'arginine, la lysine, l'ornithine, les hydroxy-prolines, la delta hydroxy-lysine, l'acide α amino adipique, les N-ω - méthyl arginines, la β hydroxy phényl alanine, la β hydroxy tyrosine, l'acide β hydroxy glutamique.

Par le terme cycloaliphatique, on entend les motifs cyclopentyle, méthyl-cyclopentyle, diméthyl-cyclopentyle, ethényl-méthyl-cyclopentyle, triméthyl-cyclopentényle, cyclopentényle, méthylcyclopentényle, cyclohéxyle ,méthylcyclohéxyle ,diméthyl-cyclohéxyle, triméthyl-cyclohéxyle, éthylcyclohéxyle , éthényl-cyclohéxyle, diméthyl-cyclohéxenyle, triméthyl-cyclohéxenyle.

Dans le cas de cycloaliphatiques substitués présentant les deux formes isomères cis et trans,on comprendra la forme pure cis, la forme pure trans, ainsi que leurs mélanges en diverses proportions.

Dans l'étape initiale, on met en réaction un dérivé, protégé à l'azote en α de l'amino acide recherché, avec l'alcool en solution dans un solvant inerte. La réaction de couplage est réalisée par un agent conventionnel, par exemple, le N.N-dicyclohexylcarbodiimide, éventuellement en présence d'une base tertiaire comme la diméthylaminopyridine.

En général, les réactifs sont employés dans les rapports de la stoechiométrie, sauf pour l'amine tertiaire qui est employée en quantitié environ égale au 1/10 de l'alcool car il est plus aisé par des lavages aqueux de retirer l'excés d'amino acide restant dans l'ester que de retirer un excés de cycloalkanol. Les réactions sont menées dans des solvants inertes vis à vis des réactifs, par exemple des hydrocarbures aliphatiques halogénés, tels que chloroforme, dichlorométhane, tétrachloroéthane ou le diméthylformamide qui présent de meilleures propriétés solvantes.

La réaction est commencée à une température voisine de 0 °C puis continuée dans les conditions de température du laboratoire pendant plusieurs heures jusqu'à ce que l'avancement de la réaction, suivi par chromatographie sur couche mince, soit suffisant. En général, une nuit de réaction est suffisante pour obtenir un rendement substantiel. L'ester formé est alors isolé par les méthodes d'extraction et de chromatographie habituelles et le groupe de protection de l'azote α est éliminé. Les conditions employées pour la déprotection dépendent évidemment du groupe bloquant utilisé, le milieu pouvant être anhydre ou aqueux et éventuellement alcalin ou acide.

Parmi les groupes protecteurs testés, le plus recommandé est le radical benzyloxycarbonyle qui peut être éliminé de façon douce par hydrogénolyse, cette hydrogénolyse étant menée dans un alcool secondaire afin d'éviter une transestérification, comme par exemple l'isopropanol ou dans l'acide acétique.

Le groupe protecteur peut également être un radical t..butyloxycarbonyle qui peut être éliminé par réaction avec l'acide trifluoroacétique à basse température afin de ne pas dégrader l'ester d'alcool primaire

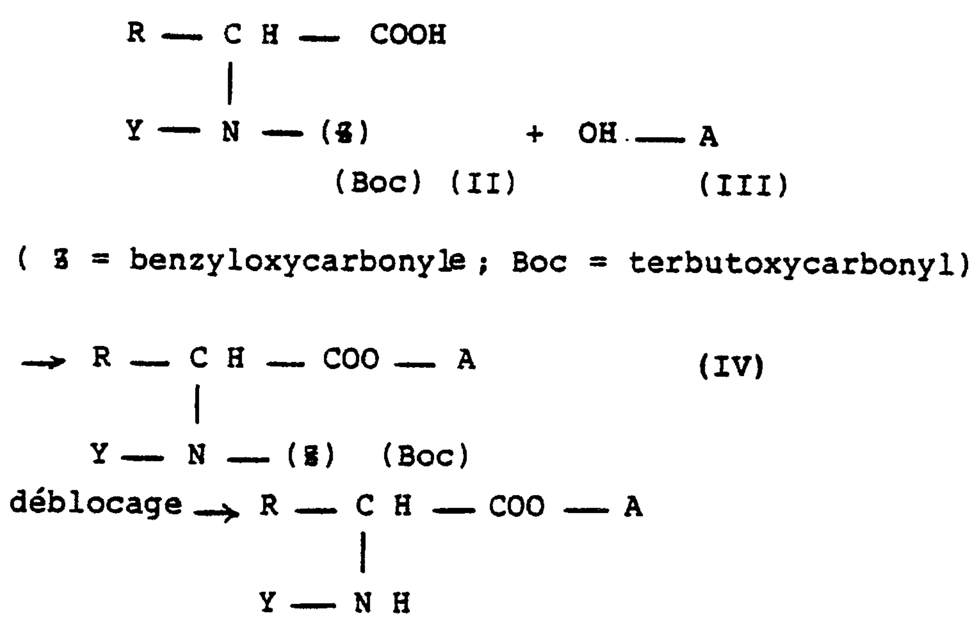
(II) (III)

( Z = benzyloxycarbonyle ; Boc = terbutoxycarbonyl)

(IV)

déblocage

Dans le cas où l'acide aminé comporte une deuxième fonction réactive acide, par exemple dans le cas des

acides glutamique, aspartique, α amino adipique, il est indispensable de bloquer comme ci-dessus la fonction aminée mais aussi la fonction ω carboxylique.

<PAR>Le blocage de cette fonction peut être réalisé selon les méthodes connues en synthèse peptidique, de préférence par un groupe ester benzylique qui sera débloqué après condensation, pendant l'hydrogénolyse du radical N-benzyloxycarbonyle(voir Methoden der Organischen Chemie, Houben Weyl, Synthese von Peptiden 15/1 p. 645).

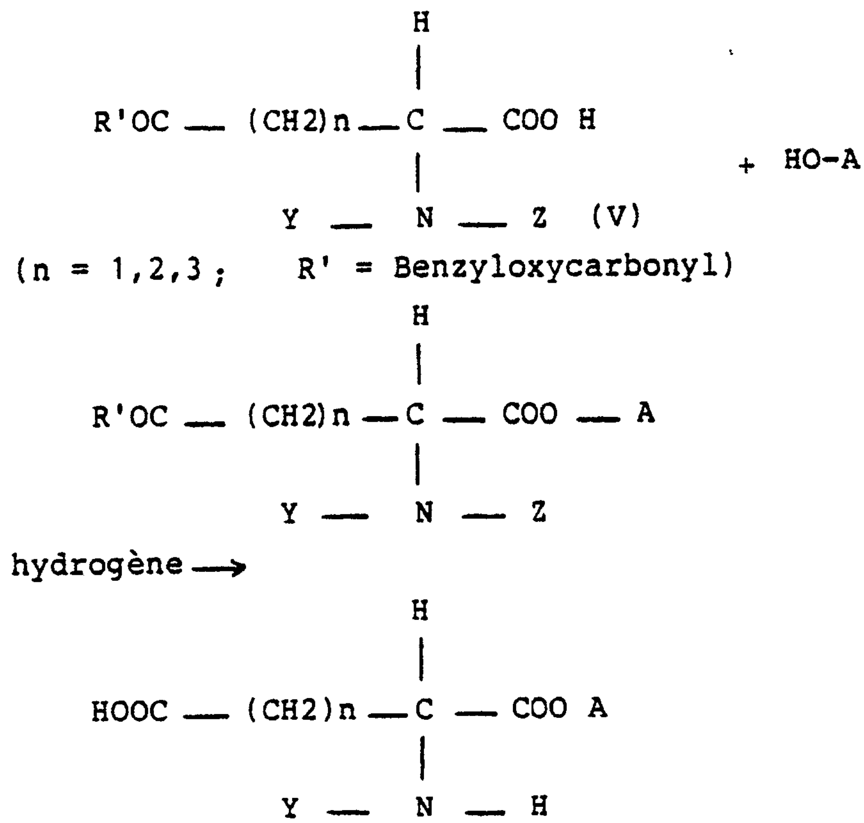

(V)

Dans le cas particulier où l'on veut obtenir l'ester en α d'un cycloalkanol, de l'acide aspartique, glutamique ou aminoadipique en conservant un dérivé simple de la fonction ω carboxylique, à savoir un ester méthylique, éthylique ou un amide primaire, il suffit de mettre en réaction un dérivé de la formule générale (V) où R′= OCH3 ou OC2H5, ou NH2, suivant le même mode opératoire ; dans ces conditions l'ester d'alkyle ou l'amide en ω de l'acide dicarboxylique n'est pas touché par l'hydrogénolyse.

Dans le cas où l'acide aminé comporte une deuxième fonction réactive -OH (alcool secondaire ou phénol) il est possible de bloquer successivement l'azote α aminé par un radical benzyloxycarbonyle et la fonction hydroxylée par un éther benzylique ou par un ester benzyloxycarbonyle dans le cas d'un OH phénolique.

Cependant, on a trouvé qu'en travaillant dans des conditions particulièrement douces (faible vitesse d'introduction de réactifs et basse température) il était seulement indispensable de bloquer l'azote porté par le carbone en 2 : ceci est particulièrement vrai avec la tyrosine et le 5-Hydroxytryptophane.

Dans le cas plus simple des polyamino acides, il suffit d'employer des quantités suffisantes de chloro formiate de benzyle pour obtenir un poly N benzyloxycarbonyle dérivé(voir la référence déjà citée, p. 528 concernant l'arginine).

L'hydrogénolyse, qui éventuellement pourra être menée en deux temps, conduit au dérivé selon la formule générale (I).

Dans le cas où dans la formule générale Y représente un radical acétyle,propionyle ou benzoyle, la protection de l'azote en α n'est plus nécessaire et la condensation du N acétyl-amino acide avec l'alcool aboutit directement au produit désiré :

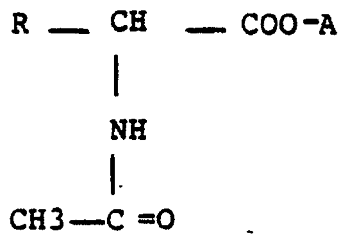

Dans le cas où dans la formule générale Y représente un radical acétyle,propionyle ou benzoyle et où R contient encore un autre radical réactif à savoir -COOH, -NH2, OH, il est nécessaire de bloquer ce dernier avant la condensation et de le débloquer ensuite selon les méthodes ci-dessus décrites.

Dans le cas où dans la formule générale (I) Y détermine un cycle avec R par exemple dans le cas de l'acide pyroglutamique

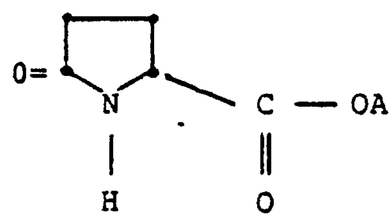

il est préférable mais non indispensable de bloquer l'azote de la pyrrolidine par un radical benzyloxycarbonyle avant la condensation de l'acide pyroglutamique. En effet, le rendement global (blocage, condensation, déblocage) est plus élevé que celui obtenu par condensation directe.

Dans le cas où l'acide aminé est bloqué par un radical peu labile par exemple quand Y = COCH3 dans la formule générale (I) ou dans le cas où R détermine avec Y un cycle pyrrolidinone 2, il est possible de réaliser l'esterification de façon habituelle dans un solvant de type hydrocarbure, par exemple, le toluène ou le xylène à température du reflux en présence d'un catalyseur acide comme l'acide sulfurique ou l'acide paratoluène sulfonique.

On sait que le cholestérol est une substance vitale pour les mammifères, dont les deux tiers sont synthétisés par le foie et un tiers est apporté par l'alimentation.

Seuls quelques pour cent de ce cholestérol lipophile sont véhiculés dans le sang, encapsulés par les lipoprotéines comme les chylomicrons, VLDL, IDL, LDL et HDL.

Le déréglement du taux de cholestérol sanguin, source, entre autres, d'athérosclérose, ne peut pas toujours être corrigé par la seule modification du régime alimentaire.

Il est donc indispensable de corriger et de réguler la production hépatique de cho térol.

Or, l'hydroxy-3 méthyl-3 glutarique CoA réductase (HMG CoA réductase ou HMGR), assurant la réduction en acide mévalonique, est le facteur principal de régulation de la production de cholestérol.

On considére en effet que la réduction HMG→acide mévalonique est l'étape clé dans la vitesse de production du cholestérol.

L'inhibition de cette enzyme peut être donc un moyen déterminant dans la réduction du taux de cholestérol sérique.

John S. BARAN and Coll (J. Med. Chem. 1985 28 597) notent l'activité des acides 3 n alkyl-3 hydroxy glutariques sur l'inhibition de HMGR.

Cette activité HMG CoA réductase peut être mesurée "in vitro" sur des cultures de fibroplastes humains selon BROWN et GOLDSTEIN (J Biol Chemist. 1974 249 789) ou encore in vivo sur le rat selon la technique de LANGDON R. (J. Lipid. Res. 1977 18 24).

Le 20-25 diazacholestérol inhibe la synthèse du cholestérol (RANNEY R.E. Proc. Soc. Exp. Biol. Med 1964 116 999) et entraine une augmentation de HMG CoA réductase pouvant atteindre 3 à 400 % de la valeur des témoins.

On peut ainsi contrôler l'activité d'un produit inhibiteur (R.J. CHORVAT H. Med. Chem. 1985 28 195).

On peut déterminer aussi une "Minimum Effective Dose" (MED) nécessaire pour abaisser par exemple de 25 % les taux HMGR sur des rats prétraités.

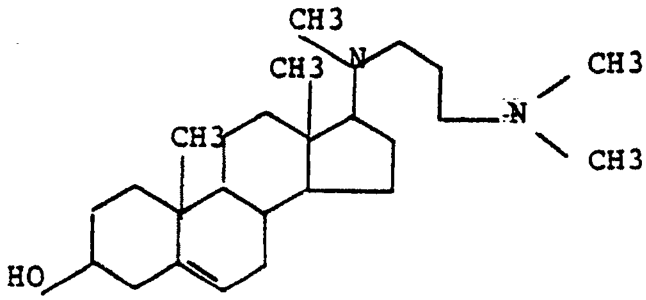

Les rats sont cependant de mauvais modèles pour l'étude de molécules capables d'inhiber la synthèse du cholestérol par inhibition de HMGR.

D'autre part, l'abaissement du taux de LDL a été consacré comme l'index thérapeutique des hypocholestérolémiants.

L'augmentation des HDL a parfois été associée aux mortalités dues aux incidents cardiovasculaires.

Il est possible à certaines drogues de modifier la synthèse du cholestérol sans faire apparaître une variation importante des taux sériques de LDL HDL.

La demanderesse a découvert que les dérivés selon la présente invention étaient des agents thérapeutiques particulièrement utiles dans la correction des dislipidémies en agissant tout particulièrement sur l'activité de HMG CoA réductase facteur principal de la synthèse du cholestérol.

Les dérivés selon la présente demande peuvent être administrés seuls ou en mélange, tels que ou à l'état de sels pharmaceutiquement acceptables, sous les différentes formes d'administration connues pour les médicaments. Ainsi de tels composés peuvent être administrés par voie entérale, parentérale, ou transcutanée, seuls ou en association avec des adjuvants ou solvants en usage dans l'industrie pharmaceutique, comme l'eau , les polyalkyleneglycols, les huiles naturelles, l'amidon, la gélatine.

Les formes pharmaceutiques solides peuvent être par exemple des comprimés, des gélules, des capsules ou suppositoires. Les formes liquides peuvent être des solutions, des suspensions ou des émulsions. La dose unitaire de la préparation pharmaceutique se situe entre 10 et 1 000 mg.

Dans les exemples non limitatif suivants, on décrit plus en détail les nouveaux composés ainsi que les procédés conformes à la présente invention pour leur préparation et les résultats des tests biochimiques ou pharmacologiques déterminant leurs activités médicamenteuses.

EXEMPLE I:-L- PYROGLUTAMATE DE TRIMETHYLCYCLOHEXANOL (trans majoritaire)

a) N benzyloxycarbonyl-L-pyroglutamate de triméthyl-3,3,5 cyclohexanol

On ajoute 34,2 g (0,13 M) d'acide N benzyloxycarbonyl-L-pyroglutamique (préparé selon Berichte 97 2434 [1964] ou Annalen 649 183 [1961]) à 150 ml de diméthylformamide, ajoute 2 g de diméthylaminopyridine puis 18,48 g (0,13 mole) de triméthyl-cyclohexanol-3,3,5 (mélange de 90 % isomère trans et 10 % isomère cis) dilué dans 50 ml de di méthylformamide. On coule ensuite en 15 minutes environ 26,8 g (0,13 mole) de dicyclohexylcarbodiimide en maintenant le milieu réactionnel à + 5 °C. On agite ensuite à température ambiante pendant 24 heures. On évapore ensuite le solvant, reprend à l'acide chlorhydrique dilué et extrait au chloroforme.

Après lavages acides, basiques et neutres de la couche organique, cette dernière est évaporée à sec et purifiée par chromatographie sur colonne de silice par un mélange de chloroforme acétone. On obtient le dérivé du titre pur à l'état d'huile épaisse avec un rendement de 65 à 70 %.

b) -L- pyroglutamate de triméthyl-3,3,5 cyclohexanol

On dissout le produit obtenu au paragraphe précédent dans 500 ml d'isopropanol et soumet à l'hydrogénation en présence de Palladium à 5 % sur charbon à température et pression ordinaires. Après filtration et évaporation du solvant, le produit est distillé sous pression réduite. Point d'ébullition : 130 °C/0,1 mm Hg.

Le produit présente un seul spot en chromatographie sur couche mince de silice (révélation-Vanilline, $SO_4H_2$) Système toluène 10, formiate d'éthyle 10, acide formique 1 : Rf = 0,45.

Système chloroforme 25, acétone 7 : Rf = 0,50.
$[\alpha]_D^{25}$ , = -6,4° (C = 1 %, DMF).
RMN (dans CDC13 par rapport à TMS)
4,2 ppm (m) 1 H en α du C = O.
2,3 ppm (m) 4 H - CH2-CH2-(cycle pyrrolidine)
3,7 ppm (m) 1H, COO-C H
entre 1 et 2 ppm (m) 7H cyclohexyl
1 ppm (s) 6H, (d) 3H, - CH 3.

EXEMPLE II - ACYLATION DIRECTE DU TRIMETHYLCYCLOHEXANOL-3,3,5 PAR L'ACIDE -L- PYROGLUTAMIQUE

On dissout 51,6 g (0,4 Mole) d'acide -L- pyroglutamique et 50 g (0,35 Mole) de triméthylcyclohexanol (enrichi en isomère trans) dans 450 ml de diméthylformamide contenant 2 g (16 millimoles) de diméthylamino-4 pyridine, puis coule 82,6 g (0,4 Mole) de dicyclohexylcarbodiimide dans 140 ml de diméthylformamide. On agite pendant 24 h, évapore, reprend à l'acide chlorhydrique dilué, extrait au chloroforme, lave au carbonate de sodium, à l'eau, sèche et distille (point d'ébullition 140-50° /0,2 mmHg) avec un rendement de 50 %.
$[\alpha]_D^{25}$ = - 6,4 ° (C = 1 %, DMF)

EXEMPLE III - ESTERIFICATION DIRECTE DU TRIMETHYL -3,3,5 CYCLOHEXANOL PAR L'ACIDE PYROGLUTAMIQUE

On dissout 26 g (0,2 Mole) d'acide pyroglutamique et 28 g (0,2 Mole) de triméthyl cyclohexanol-3,3,5 (isomère enrichi à 90 % en trans) dans 200 ml de diméthyl formamide et 500 ml de toluène contenant 2 ml d'acide sulfurique On porte à reflux sous agitation vive en éliminant progressivement l'eau formée, pendant 20 heures. Après évaporation du solvant, reprise au chloroforme et lavages à l'eau, on évapore sous vide le solvant, puis le triméthyl cyclohexanol non réagi et on distille le dérivé du titre à 160/170° /0,7 mmHg. Le rendement est en moyenne de 20 %.

EXEMPLE IV - PYROGLUTAMATE DU TRIMETHYL-3,3,5 cyclohexanol (cis) majoritaire

On opère dans les conditions de l'exemple II en utilisant du triméthyl cyclohexanol-3,3,5 enrichi à 90 % en son isomère cis.

Le produit est chromatographié sur gel de Silice par un mélange de chloroforme et d'éthanol.

On obtient après évaporation du solvant, 25 % du dérivé du titre sous forme d'une huile épaisse.

En CCM, le produit présente un seul spot dans les deux systèmes de solvant décrits dans l'exemple I, le Rf est identique à celui de l'isomère trans (enrichi)
$[\alpha]_D^{25}$ = -1,5 ° (C = 1 %, DMF)
En infra rouge : bandes caractéristiques :
3300 -3200 cm-1 massif : 2980 - 2820 cm-1, CH2 - CH3 :
1730 - 1700 cm-1 (FF).

EXEMPLE V - L-GLUTAMINE ESTER DU TRIMETHYL-3,3,5 CYCLOHEXANOL, CHLORHYDRATE

a) N-benzyloxycarbonyl-L-glutamine ester du triméthyl-3,3,5 cyclohexanol

On ajoute 16,10 g (57,5 millimoles) de l' α N-benzylo xycarbonyl-L- glutamine (préparée selon la technique générale décrite dans Methoden der Organischen Chemie - Houben Weyl - Synthese von Peptiden 15 -1- p. 701 - Georg Thieme Verlag) à 100 ml de diméthylformamide puis introduit 7,11 g (50 millimoles) de triméthylcyclohexanol-3,3,5 (mélange de 90 % d'isomère trans et 10 % de cis) et 0,92 g (7,5 millimoles) de diméthylamino-4-pyridine. On refroidit entre 0 et + 5° et introduit lentement 11,9 g (57,5 millimoles) de dicyclohexylcarbodiimide.

On agite ensuite pendant 24 heures à température ordinaire et évapore à sec. On reprend le résidu à l'acide chlorhydrique dilué et au chloroforme en traitant comme dans l'exemple I. Le produit est purifié par chromatographie sur colonne de silice par un mélange chloroforme isopropanol.

On obtient ainsi 70 % du dérivé du titre à l'état pur présentant en CCM sur silice dans le système solvant chloroforme 30 isopropanol 2 un Rf = 0,30.

Si la CCM est effectuée dans le système toluène 10/formiate d'éthyle 10/acide formique 1, on peut distinguer les spots des deux esters iomères trans et cis respectivement à Rf = 0,52 et Rf = 0,47.

Par chromatographie sur colonne de silice par un mélange chloroforme isopropanol, on peut séparer la majeure partie de l'ester trans de Rf = 0,52.

b) -L- γ amido glutamate du trimethyl-3,3,5 cyclohexanol (trans)

On soumet à l'hydrogénolyse le produit précédemment obtenu, dans l'isopropanol en présence de Palladium sur charbon. En fin de réaction, on filtre, évapore et obtient le dérivé du titre sous forme d'huile épaisse présentant en CCM dans le système n butanol 8/acide acétique 1/eau 1/ un seul spot de Rf = 0,37.

Par action de l'éther chlorhydrique, on peut faire cristalliser le -L- γ amido glutamate de trans trimethyl-3,3,5 cyclohexanol chlorohydrate de pF = 125/129 °C.

EXEMPLE VI - -L- α GLUTAMATE DU TRIMETHYL-3,3,5 CYCLOHEXANOL TRANS CHLORHYDRATE

a) N-benzyloxycarbonyl- γ benzyl α -L-glutamate de trimethyl-3,3,5 cyclohexanol

On ajoute 37,1 g (0,1 M) d'acide N-benzyloxycarbonyl-γ benzyl-L-glutamique préparé selon KLIEGER et GIBIAN (Annalen 655 - 195 - 1962) à 150 ml de diméthylformamide contenant 1,5 g de diméthylamino-4 pyridine, puis introduit 14,2 g (0,1 Mole) de triméthyl-3,3,5 cyclohexanol.

On refroidit sous agitation entre 0 et + 5 °C et coule 20,6 g (0,1 Mole) de dicyclohexyl carbodiimide dilué dans 200 ml de DMF en maintenant la température inférieure à + 5 °C. On agite ensuite à température ordinaire jusqu'à accomplissement de la réaction, détecté par CCM.

On évapore le solvant puis traite comme dans les essais précédents. On obtient après chromatographie sur colonne de silice, le dérivé du titre à l'état pur avec un rendement de 55 % et présentant en CCM sur silice dans le système solvant chloroforme 30 isopropanol 2 un seul spot de Rf = 0,35. Par contre en CCM dans le système toluène 10, formiate d'éthyle 10, acide formique 1, on distingue le Rf = 0,55 Ester du triméthyl cyclohexanol-3,3,5 forme trans et le Rf 0,45 Ester de la forme cis. L'ester est donc chromatographié sur colonne de silice pour séparer l'ester N-Z- γ (OBz) glutamique du transtriméthyl cyclohexanol présentant un seul spot à 0,55.

b) α -L-glutamate du triméthyl-3,3,5 cyclohexanol (trans) chlorhydrate

On soumet le produit obtenu au paragraphe précédent à l'hydrogénolyse dans l'isopropanol dans les conditions ordinaires de température et de pression en présence de Palladium sur charbon. On obtient ainsi le dérivé du titre à l'état pur sous forme d'huile amorphe présentant en CCM sur silice un seul spot de Rf = 0,37 dans le système : n butanol 10, acide acétique 1, eau 1. On peut convertir ce produit en son chlorhydrate au moyen de l'éther chlorhydrique et l'on obtient ainsi un produit bien cristallisé de pF 95° / 100 °C.

EXEMPLE VII - N ACETYL-L- α ASPARTATE DU TRIMETHYL-3,3,5 CYCLOHEXANOL SEL DE MAGNESIUM

a) N acétyl β benzyl-L- α aspartate du triméthyl-3,3,5 cyclohexanol

On ajoute 26,5 g (0,1 Mole) de N acétyl β benzyl -L-aspartique acide (préparé selon J. of Pharmaceuti-

cal Sciences 52 - 9 - 1963) à 150 ml de diméthylformamide contenant, 1,5 g de diméthylamino-4 pyridine puis introduit 14,2 g (0,1 Mole) de triméthyl-3,3,5 cyclohexanol. On refroidit entre 0 et + 5 °C et coule 20,6 g (0,1 Mole) de dicyclohexyl carbodiimide dilué dans 200 ml de DMF. On traite ensuite comme à l'exemple VI-a. On obtient le dérivé du titre mélange des deux isomères cis-trans présentant un seul spot de Rf 0,25 dans le système chloroforme 30/isopropanol 2-.

b) N acetyl-L- α aspartate du triméthyl-3,3,5 cyclohexanol sel de magnesium

On soumet à l'hydrogénolyse le produit obtenu au paragraph précédent dans les conditions de l'exemple VI-b. Après traitement, on obtient le N acetyl-L-aspartate de triméthylcyclohexanol-3,3,5 à l'état d'huile épaisse. Le produit est mis en suspension aqueuse et traité par de la magnésie jusqu'à obtention d'un pH de 6,0/7,0. Après filtration, on évapore sous vide à basse température et obtient le dérivé du titre à l'état de sel de magnésium.

En traitant l'acide - L - pyroglutamique avec les quantités stoechiométriques de divers alcools cycloaliphatiques substitués ou non comme le cyclohexanol, le diméthyl-3,5 cyclohexanol (mélange cis-trans), le triméthyl-3,5,5 cyclohexèn-2 ol-1, le méthyl-1 cyclopentanol, le terpinène-4 ol, le terpinéol et ce dans les conditions décrites dans l'exemple III, on obtient les dérivés ci-après :

| -L-Pyroglutamate du | Pt. ébullition |
|---|---|
| Exemple VIII cyclohexanol | 175°C/18mm Hg |
| Exemple IX -3,5 diméthyl cyclohexanol | 190/200°C/18 mm |
| Exemple X -2,6 diméthyl cyclohexanol | 190/200°C/18mm |
| Exemple XI -3,5,5 triméthyl 2 cyclohexénol-1 | 140°C /0,2 |
| Exemple XII -1 méthyl cyclopentanol | 140°C/18 |
| Exemple XIII Terpinène-4.ol | 120°C /0,1 |
| Exemple XIV Terpinéol | 115/120°C/0,1 |

EXEMPLE XV - ACTIVITE SUR L'HYPERLIPEMIE EXPERIMENTALE INDUITE AU TRITON

On traite des groupes de 10 rats à jeun d'environ 200 g par injection intrapéritonéale de Triton à la dose de 300 mg/kg puis administre immédiatement les produits par voie orale.
Après 18 heures, on procède à la détermination du cholestérol et des triglycérides. Les résultats figurent dans le tableau suivant :

| Traitement | Dose mg/kg | (g/l)Taux de triglycéride | | (g/l)Taux de cholestérol | |
|---|---|---|---|---|---|
| Témoin sans triton | | 0,62 ± | 0,12 | 2,16 ± | 0,30 |
| Témoin Triton | | 6,41 | 2,60 | 5,04 | 1,04 |
| Ac. nicotinique réf. | 500 | 1,41 | 0,31 | 5,00 | 1,20 |
| Produit de l'ex I | 150 | 0,93 | 0,38 | 1,95 | 0,36 |
| Produit de l'ex I | 50 | 1,46 | 0,47 | 2,80 | 0,60 |
| Produit de l'ex III | 150 | 1,10 | 0,30 | 2,00 | 0,33 |
| Produit de l'ex IV | 150 | 0,85 | 0,43 | 2,15 | 0,61 |
| Produit de l'ex V | 150 | 1,15 | 0,27 | 1,95 | 0,20 |
| Produit de l'ex VI | 150 | 2,00 | 0,30 | 2,50 | 0,30 |
| Produit de l'ex VII | 150 | 2,70 | 0,40 | 3,00 | 0,55 |
| Produit de l'ex IX | 150 | 1,30 | 0,33 | 2,10 | 0,20 |
| Produit de l'ex XI | 150 | 1,92 | 0,41 | 2,00 | 0,33 |
| Produit de l'ex XIII | 150 | 1,20 | 0,34 | 1,90 | 0,52 |
| Produit de l'ex XIV | 150 | 1,10 | 0,19 | 2,12 | 0,31 |

EXEMPLE XVI - ACTIVITE HYPOLIPIDEMIANTE CHEZ LE RAT HYPERLIPIDEMIE A L'HUILE D'OLIVE

Un lot de rats est soumis à un régime hyperlipidémiant athérogène enrichi à l'huile d'olive et les animaux sont ensuite traités p. os.

Cholestérol et triglycérides sont ensuite déterminés comme dans l'exemple précédent.

On a évidemment dosé cholestérol et triglycérides sur des animaux provenant de la même sélection mais n'ayant pas été prétraités au Triton (Témoin) et sur des animaux pré-traités au Triton (témoin hyperlip.)

| Traitement | Dose mg/kg | (g/l)Taux de triglycéride | (g/l)Taux de cholestérol |
|---|---|---|---|
| Témoin | | 0,61 ± 0,10 | 2,38 ± 0,19 |
| Témoin hyperlip. | | 2,45 0,43 | 2,71 0,92 |
| Produit de l'ex I | 100 | 0,80 0,29 | 1,50 0,47 |
| Produit de l'ex I | 25 | 1,39 0,19 | 1,73 0,24 |
| Produit de l'ex III | 100 | 1,01 0,37 | 1,47 0,21 |
| Produit de l'ex IV | 100 | 0,80 0,42 | 1,47 0,37 |
| Produit de l'ex V | 100 | 0,92 0,18 | 1,62 0,42 |

EXEMPLE XVII - DETERMINATION DE L'ACTIVITE INHIBITRICE DES COMPOSES VIS-A-VIS DE L'HYDROXY-3 METHYL-3 GLUTARYL COENZYME A REDUCTASE

La méthode utilisée est celle décrite par Shefer et Coll. (J. Lipid. Res 1972, 13 , 402) voir également Stokker (J. Med. Chem. 1985 (28) 357).
Les doses inhibitrices 50 sont représentées dans le tableau suivant :

| TRAITEMENT | $IC_{50}$ µM |
|---|---|
| Produit de l'exemple I | 0,10 |
| Produit de l'exemple IV | 0,15 |
| Produit de l'exemple V | 0,08 |
| Produit de l'exemple VI | 0,12 |
| Produit de l'exemple VII | 0,50 |

| TRAITEMENT | IC50 µM |
|---|---|
| Produit de l'exemple VIII | 0,55 |
| Produit de l'exemple IX | 0,40 |
| Produit de l'exemple X | 0,40 |
| Produit de l'exemple XI | 0,13 |
| Produit de l'exemple XII | 0,60 |
| Produit de l'exemple XIII | 0,21 |
| Produit de l'exemple XIV | 0,30 |

**Revendications**

1. Esters d'aminoacides d'alcools cycloaliphatiques répondant à la formule générale (I) :

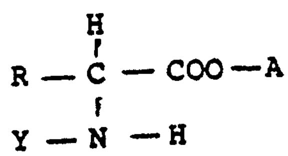

dans laquelle :
R représente le reste d'un α aminoacide naturel ou d'un dérivé simple de ce dernier dans le cas des aminoacides dicarboxyliques, tel que l'ester méthylique, l'ester éthylique ou un amide primaire,
Y est l'hydrogène, un radical acétyle, propionyle, benzoyle,
ou bien représente avec R un cycle pyrrolidinone 2 déterminant dans la formule générale un ester pyroglutamique,
A représente un motif cycloaliphatique choisi dans le groupe composé de cyclohexyle, cyclohéxenyle, cyclopentyle, cyclopentenyle toujours substitué par un ou plusieurs radicaux choisis parmi les groupes : méthyle, éthyle, méthylène, éthylène, éthényle.

2. Composés suivant la revendication 1 où le motif A est un groupement cyclohexyle, substitué par un ou plusieurs radicaux méthyles.

3. Composés suivant la revendication 1 où le motif A est un groupement cyclopentyle, substitué par un ou plusieurs radicaux méthyles.

4. Composés suivant les revendications 1,2,3 où l'acide aminé est l'acide pyroglutamique.

5. L'ester pyroglutamique du triméthyl cyclohexanol-3,3,5

6. Composés suivant les revendications 1,2,4 où le triméthyl -3.3.5 cyclohexanol est sous une forme enrichie à plus de 90 % en isomère cis ou isomère trans.

7. Composés suivant les revendications 1,2,3,4,5 où le l'acide pyroglutamique est de configuration L.

8. Procédé de préparation des dérivés selon la revendication 1 selon lequel l'acide aminé dont les fonctions réactives non intéressées ont été préalablement protégées, est mis en réaction dans un

solvant inerte avec un cycloalkanol en présence de dicyclohexyl-carbodiimide, en présence d'une quantité catalytique de diméthylamino pyridine à température ordinaire, purifié par chromatographie ou distillation, puis libéré de ses groupes protecteurs, de préférence des groupes benzyloxycarbonyle. par hydrogénolyse.

**9.** Médicament contenant au moins un des dérivés selon les revendications 1 à 7, à l'état pur ou à l'état de sels avec des acides ou des bases pharmaceutiquement acceptables, destiné à combattre les dérèglements de la lipidémie chez l'homme ou l'animal.

Revendications pour les Etats contractants suivants : AT, GR, ES

**1.** Procédé de préparation d'esters d'aminoacides d'alcools cycloaliphatiques répondant à la formule générale (I)

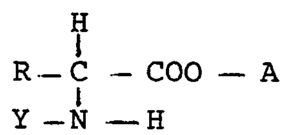

dans laquelle :

R représente le reste d'un α aminoacide naturel ou d'un dérivé simple de ce dernier dans le cas des aminoacides dicarboxyliques, tel que l'ester méthylique, l'ester éthylique ou un amide primaire,

Y est l'hydrogène, un radical acétyle, propionyle, benzoyle, ou bien représente avec R un cycle pyrrolidinone 2 déterminant dans la formule générale un ester pyroglutamique,

A représente un motif cycloaliphatique choisi dans le groupe composé de cyclohexyle, cyclohéxenyle, cyclopentyle, cyclopentenyle toujours substitué par un ou plusieurs radicaux choisis parmi les groupes : méthyle, éthyle, méthylène, éthylène, éthényle,

caractérisé en ce que l'alide aminé dont les fonctions réactives non intéressées ont été préalablement protégées, est mis en réaction dans un solvant inerte avec un cycloalkanol en présence de dicyclohexyl-carbodiimide, en présence d'une quantité catalytique de diméthylaminopyridine à température ordinaire, purifiée par chromatographie ou distillation, puis libéré de ses groupes protecteurs, de préférence des groupes benzyloxycarbonyle, par hydrogénolyse.

**2.** Procédé selon la Revendication 1, caractérisé en ce que le motif A est un groupement cyclohexyle, substitué par un ou plusieurs radicaux méthyles.

**3.** Procédé selon la Revendication 1, caractérisé en ce que le motif A est un groupement cyclopentyle, substitué par un ou plusieurs radicaux méthyles.

**4.** Procédé selon l'une quelconque des Revendications 1 à 3, caractérisé en ce que l'acide aminé est l'acide pyroglutamique.

**5.** Procédé selon les Revendications 1, 2 ou 4, caractérisé en ce que le triméthyl-3.3.5 cyclohexanol est sous une forme enrichie à plus de 90% en isomère cis ou isomère trans.

**6.** Procédé selon les Revendications 1 à 4, caractérisé en ce que l'acide pyroglutamique est de configuration L.

**Claims**

**1.** An ester of an amino acid and a cycloaliphatic alcohol, of general formula (I):

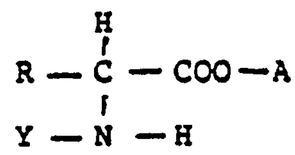

in which:
R is the radical of a natural α-amino acid or of a simple derivative of the latter in the case of dicarboxylic amino acids, such as the methyl ester, the ethyl ester or a primary amide,
Y is hydrogen or an acetyl, propionyl or benzoyl radical or, with R, is a pyrrolidin-2-one ring forming a pyroglutamic ester in the general formula, and
A is a cycloaliphatic unit selected from the group consisting of cyclohexyl, cyclohexenyl, cyclopentyl and cyclopentenyl, which is always substituted by one or more radicals selected from methyl, ethyl, methylene, ethylene and ethenyl groups.

2. A compound according to Claim 1 in which the unit A is a cyclohexyl group substituted by one or more methyl radicals.

3. A compound according to Claim 1 in which the unit A is a cyclopentyl group substituted by one or more methyl radicals.

4. A compound according to Claims 1, 2 and 3 in which the amino acid is pyroglutamic acid.

5. The pyroglutamic ester of 3,3,5-trimethylcyclohexanol.

6. A compound according to Claims 1, 2 and 4 in which the 3,3,5-trimethylcyclohexanol is in a form enriched to more than 90% in cis isomer or trans isomer.

7. A compound according to Claims 1, 2, 3, 4 and 5 in which the pyroglutamic acid is in the L configuration.

8. A method of preparing the derivatives according to Claim 1, wherein the amino acid, whose non-participatory reactive groups have been protected beforehand, is reacted with a cycloalkanol in an inert solvent, in the presence of dicyclohexylcarbodiimide and in the presence of a catalytic amount of dimethylaminopyridine, at ordinary temperature, the product is purified by chromatography or distillation and its protecting groups, preferably benzyloxycarbonyl groups, are then removed by hydrogenolysis.

9. A drug containing at least one of the derivatives according to Claims 1 to 7, in the pure state or in the form of salts with pharmaceutically acceptable acids or bases, for combating the disorders of lipidaemia in humans or animals.

Claims for the following Contracting States : AT, ES, GR.

1. A method of preparing esters of amino acids and cycloaliphatic alcohols, of general formula (I):

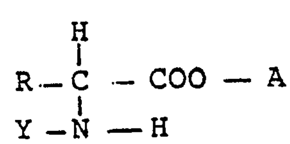

in which:
R is the radical of a natural α-amino acid or of a simple derivative of the latter in the case of dicarboxylic amino acids, such as the methyl ester, the ethyl ester or a primary amide,
Y is hydrogen or an acetyl, propionyl or benzoyl radical or, with R, is a pyrrolidin-2-one ring forming a

pyroglutamic ester in the general formula, and
A is a cycloaliphatic unit selected from the group consisting of cyclohexyl, cyclohexenyl, cyclopentyl and cyclopentenyl, which is always substituted by one or more radicals selected from methyl, ethyl, methylene, ethylene and ethenyl groups,
characterized in that the amino acid, whose non-participatory reactive groups have been protected beforehand, is reacted with a cycloalkanol in an inert solvent, in the presence of dicyclohexylcarbodiimide and in the presence of a catalytic amount of dimethylaminopyridine, at ordinary temperature, the product is purified by chromatography or distillation and its protecting groups, preferably benzyloxycarbonyl groups, are then removed by hydrogenolysis.

2. A method according to Claim 1, characterized in that the unit A is a cyclohexyl group substituted by one or more methyl radicals.

3. A method according to Claim 1, characterized in that the unit A is a cyclopentyl group substituted by one or more methyl radicals.

4. A method according to any one of Claims 1 to 3, characterized in that the amino acid is pyroglutamic acid.

5. A method according to Claim 1, 2 or 4, characterized in that the 3,3,5-trimethylcyclohexanol is in a form enriched to more than 90% in cis isomer or trans isomer.

6. A method according to Claims 1 to 4, characterized in that the pyroglutamic acid is in the L configuration.

## Ansprüche

1. Aminosäureester von cycloaliphatischen Alkoholen gemäß der allgemeinen Formel (I)

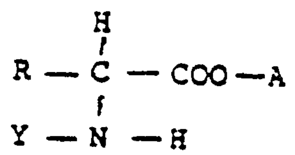

worin R für einen natürlichen α-Aminosäurerest oder ein einfaches Derivat davon im Falle von dicarboxylischen Aminosäuren steht, wie Methylester, Ethylester oder primäres Amid, Y für Wasserstoff, einen Acetylrest, Propionylrest, Benzoylrest steht,
oder mit R einen 2-Pyrrolidinonring, der in der allgemeinen Formel einen Pyroglutaminsäureester darstellt, bildet, A für eine cycloaliphatische Gruppe, ausgewählt aus der Gruppe Cyclohexyl, Cyclohexenyl, Cyclopentyl, Cyclopentenyl, immer durch einen oder mehrere Reste, ausgewählt unter den Gruppen Methyl, Ethyl, Methylen, Ethylen, Ethenyl, substituiert, steht.

2. Verbindungen nach Anspruch 1, **dadurch gekennzeichnet,** daß der Rest A eine Cyclohexylgruppe, substituiert durch einen oder mehrere Methylreste, ist.

3. Verbindungen nach Anspruch 1, **dadurch gekennzeichnet,** daß der Rest A eine Cyclopentylgruppe, substituiert durch einen oder mehrere Methylreste, ist.

4. Verbindungen nach einem der Ansprüche 1, 2, 3, **dadurch gekennzeichnet,** daß die Aminosäure Pyroglutaminsäure ist.

5. 3,3,5-Trimethylcyclohexanol-Pyroglutaminsäureester.

6. Verbindungen nach den Ansprüchen 1, 2, 4, **dadurch gekennzeichnet,** daß 3, 3, 5-Trimethylcyclohexanol in einer Form, die zu mehr als 90% an cis-Isomeren oder trans-Isomeren angereichert ist, vorliegt.

7. Verbindungen nach den Ansprüchen 1, 2, 3, 4, 5, **dadurch gekennzeichnet,** daß die Pyroglutaminsäure in L-Konfiguration vorliegt.

8. Verfahren zur Herstellung der Derivate nach Anspruch 1. **dadurch gekennzeichnet,** daß man die Aminosäure, deren nicht-interessierenden, reaktiven Funktionen zuvor geschützt worden sind, in einem inerten Lösungsmittel mit einem Cycloalkanol in Gegenwart von Dicyclohexylcarbodiimid, in Gegenwart einer katalytischen Menge Dimethylaminopyridin bei üblicher Temperatur umsetzt, durch Chromatografie oder Destillation reinigt, anschließend von den Schutzgruppen, bevorzugt den Benzyloxycarbonylgruppen, durch Hydrogenolyse befreit.

9. Medikament, enthaltend mindestens eines der Derivate nach den Ansprüchen 1 bis 7 in reiner Form oder als Salz mit pharmazeutisch annehmbaren Säuren oder Basen, zur Bekämpfung von Fehlregulationen der Lipoidämie bei Menschen oder beim Tier.

Patentansprüche für folgende Vertragsstaaten : AT, GR, ES.

1. Verfahren zur Herstellung von Aminosäureester von cycloaliphatischen Alkoholen gemäß der allgemeinen Formel (I)

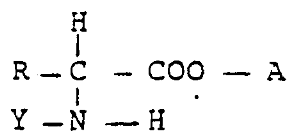

worin
R für einen natürlichen α-Aminosäurerest oder ein einfaches Derivat davon im Falle von dicarboxylische Aminosäuren steht, wie Methylester, Ethylester oder primäres Amid,
Y für Wasserstoff, einen Acetylrest, Propionylrest, Benzoylrest steht, oder mit R einen 2-Pyrrolidinonring bildet, der in der allgemeinen Formel einen Pyroglutaminsäureester darstellt,
A für eine cycloaliphatische Gruppe, ausgewählt aus der Gruppe Cyclohexyl, Cyclohexenyl, Cyclopentyl, Cyclopentenyl, immer durch einen oder mehrere Reste, ausgewählt aus den Gruppen Methyl, Ethyl, Methylen, Ethylen, Ethenyl, steht, **dadurch gekennzeichnet,** daß man die Aminosäure, deren nicht-interessierenden, reaktiven Funktionen zuvor geschützt worden sind, in einem inerten Lösungsmittel mit einem Cycloalkanol in Gegenwart von Dicyclohexylcarbodiimid, in Gegenwart einer katalytischen Menge Dimethylaminopyridin bei üblicher Temperatur umsetzt, durch Chromatografie oder Destillation reinigt, anschließend von den Schutzgruppen, bevorzugt den Benzyloxycarbonylgruppen, durch Hydrogenolyse befreit.

2. Verfahren nach Anspruch 1, **dadurch gekennzeichnet,** daß der Rest A eine Cyclohexylgruppe, substituiert durch einen oder mehrere Methylreste ist.

3. Verfahren nach Anspruch 1, **dadurch gekennzeichnet,** daß die Gruppe A eine Cyclopentylgruppe, substituiert durch einen oder mehrere Methylreste ist.

4. Verfahren nach einem der Ansprüche 1 bis 3, **dadurch gekennzeichnet,** daß die Aminosäure Pyroglutaminsäure ist.

5. Verfahren nach den Ansprüchen 1, 2 oder 4, **dadurch gekennzeichnet,** daß 3,3,5-Trimethylcyclohexanol in einer Form angereichert zu mehr als 90% an cis-Isomeren oder trans-Isomeren vorliegt.

6. Verfahren nach den Ansprüchen 1 bis 4, **dadurch gekennzeichnet,** daß die Pyroglutaminsäure in L-Konfiguration vorliegt.